# FASCICULE DE BREVET EUROPEEN

(11) **EP 1 334 705 B1**
(45) Date de publication et mention de la délivrance du brevet: **01.03.2006**
(21) Numéro de dépôt: 02356254.9
(22) Date de dépôt: 06.12.2002
(51) Int. Cl.: A61F 5/37

(54) **Dispositif de rééducation scapulo-humérale**
Schulterblatt/Oberarm-Rehabilitationsgerät
Scapulo-humeral rehabilitation device

(30) Priorité: 06.12.2001 FR 0115795
(43) Date de publication de la demande: 13.08.2003
(73) Titulaire: Travers, M. Vincent, 69002 Lyon (FR)
(72) Inventeur: Travers, M. Vincent, 69002 Lyon (FR)
(74) Mandataire: Guerre, Dominique

(56) Documents cités:
- EP-A- 0 264 303
- DE-A- 3 612 426
- DE-U- 8 912 344
- US-A- 5 538 499

## Description

La présente invention concerne un dispositif de rééducation de l'articulation scapulo-humérale.

La chirurgie réparatrice de la coiffe des rotateurs et de la synthèse de trochiter est en pleine expansion.

L'un des facteurs essentiels de réussite de cette chirurgie réside dans la rééducation post-opératoire.

Celle-ci doit, en effet, présenter un caractère dynamique pour éviter d'éventuelles raideur ou atrophie musculaire, tout en garantissant la sécurité de l'opération en prévenant une éventuelle rupture ligamentaire ou osseuse.

De façon traditionnelle un patient ayant subi une intervention chirurgicale concernant les articulations des membres supérieurs et, notamment, de l'articulation scapulo-humérale, revêt une attelle immobilisant son membre supérieur.

Cette attelle maintient le bras et le coude contre le corps du patient. L'attelle peut être retirée temporairement avec l'aide d'un kinésithérapeute pour pratiquer une mobilisation des membres. L'attelle est ensuite de nouveau mise en place sur le patient. On comprend donc que dans la période post-opératoire, le membre est majoritairement maintenu dans une position statique. Les phases de travail dynamique sont très minoritaires et ne peuvent être effectuées qu'en milieu médical ou paramédical, leur effet est donc faible.

Il existe également des attelles d'abduction qui réalisent une immobilisation du bras dans une position dans laquelle le membre est écarté de l'axe médian du corps. Si ces attelles favorisent la cicatrisation tendineuse et la consolidation osseuse grâce à l'abduction qu'elles procurent, elles présentent le défaut de maintenir le membre statiquement avec un angle d'abduction fixe.

Le document US-A-5538499 montre ainsi un dispositif orthopédique ayant un corset sur lequel est articulé un support brachial. L'articulation du support brachial sur le corset met en oeuvre un système complexe de butées de réglage de l'abduction nécessitant notamment l'utilisation d'outil.

L'invention a pour but de résoudre ces inconvénients.

L'invention a, notamment, pour but de proposer un dispositif de rééducation scapulo-humérale qui, tout en garantissant la cicatrisation ou consolidation de l'articulation d'un membre supérieur, autorise une mobilisation contrôlée de ce dernier.

A cet effet, ce dispositif de rééducation scapulo-humérale selon l'invention présente essentiellement les caractéristiques visées par la revendication 1.

Grâce à ses possibilités de réglage, le dispositif peut s'adapter de manière optimale à un patient et à la nature de l'intervention qu'il a subie. En effet, le dispositif autorise un réglage de l'angle d'abduction initial et de l'arc de mobilité qui varie d'une valeur nulle à une valeur dont l'amplitude augmente au fil de la cicatrisation.

Selon un mode de réalisation particulièrement avantageux, les points de fixation du vérin sont réglables respectivement verticalement par rapport au corset et longitudinalement par rapport au support brachial.

En agissant sur les points de fixation du vérin, l'angle d'abduction, c'est-à-dire l'angle que fait le support brachial par rapport au corset peut être aisément ajusté en fonction de caractéristiques morphologique et pathologique d'un patient.

De manière avantageuse, le vérin comprend un cylindre dans lequel coulisse un piston, le cylindre présentant des perçages dans lesquels est susceptible d'être engagé un ergot formant butée pour le piston.

L'engagement de l'ergot permet de régler la course du vérin et donc l'amplitude de l'arc de mobilité du membre supérieur.

Selon une caractéristique avantageuse, l'engagement de l'ergot dans le perçage du cylindre le plus éloigné de l'extrémité du vérin annule la course du vérin.

De préférence, un cavalier en matière élastique susceptible d'être encliqueté sur le cylindre supporte l'ergot.

Grâce à ce cavalier, la course du vérin et donc, l'arc de mobilité, peut être très facilement sélectionnée.

Selon une possibilité, les moyens de fixation du vérin permettent son interchangeabilité.

L'interchangeabilité du vérin au profit d'un vérin présentant une raideur différente permet au fil de la rééducation de modifier la force que doit exercer le membre supérieur, pour faire pivoter le support brachial.

De façon avantageuse, une charnière assure l'articulation entre le support brachial et un montant vertical fixé au corset.

En outre, un support anté-brachial est articulé sur l'extrémité distale du support brachial.

De façon avantageuse, le corset comprend une plaque d'appui pubien, deux plaques d'appui latéral, une plaque d'appui sternal et une plaque d'appui huméral reliées par des barres de liaison.

De plus, un appui dorsal est relié par une ceinture souple de manière fixe à l'un des appuis latéraux et de manière amovible à l'autre appui latéral.

Selon une possibilité, une boule est disposée à l'extrémité distale du support anté-brachial.

Pour sa bonne compréhension, l'invention est décrite en référence au dessin annexé représentant, à titre d'exemple non limitatif, un dispositif de rééducation scapulo-humérale selon celle-ci.
La figure 1 est une vue en perspective du dispositif.
La figure 2 est une vue de côté du dispositif.
La figure 3 est une vue en coupe selon III-III de la figure 2.

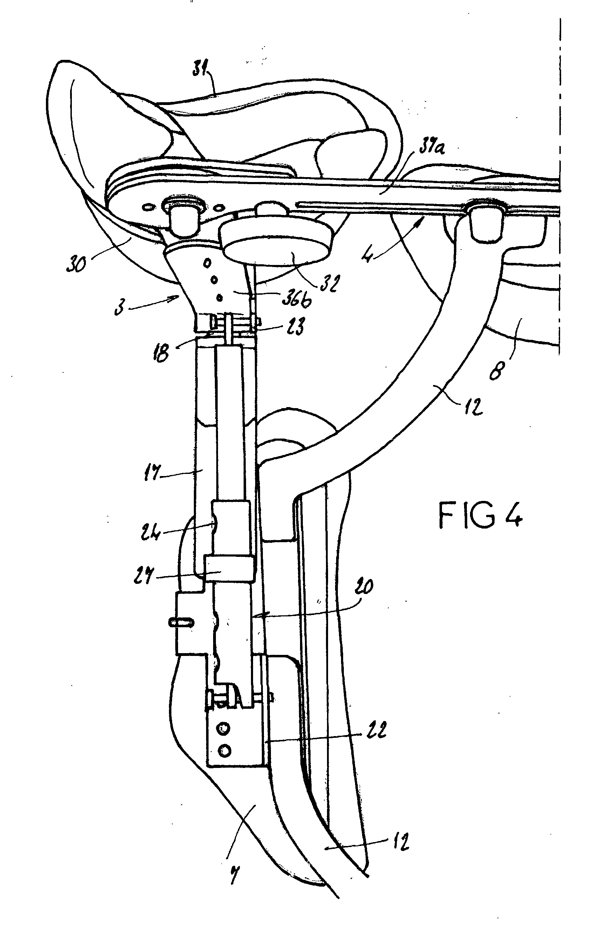

La figure 4 est une vue en face d'un support brachial.

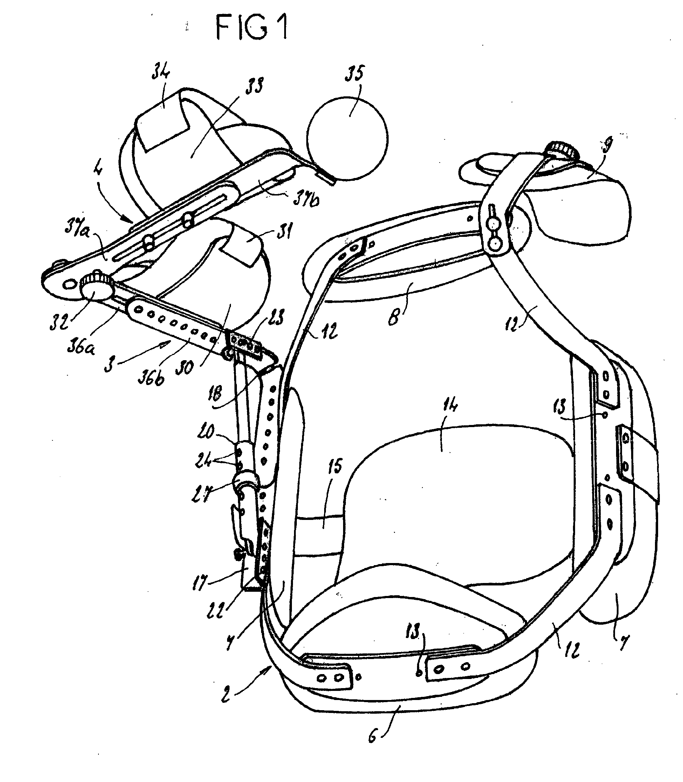

Comme cela apparaît à la figure 1, le dispositif de rééducation comprend un corset 2 sur lequel est fixé un support brachial 3 prolongé par un support anté-brachial 4.

Le corset 2 destiné à être positionné sur le tronc d'un patient comprend plusieurs éléments d'appui sur celui-ci. Il s'agit d'une plaque d'appui pubien 6, de deux plaques d'appui latéral 7, d'une plaque d'appui sternal 8 et d'une plaque d'appui huméral 9. Ces plaques d'appui sont reliées entre elles par des barres métalliques 12. Pour une parfaite adaptation à chaque morphotype, les points d'attache des barres sur les plaques d'appui sont ajustables grâce à plusieurs perçages 13 ménagés dans les plaques d'appui 6, 7, 8, 9.

Une plaque d'appui dorsal 14 achève le maintien du corset sur le tronc. La plaque d'appui dorsal 14 est reliée par une ceinture souple 15, de manière fixe à l'une des plaques d'appui latéral 7 et de manière amovible à l'autre plaque d'appui latéral 7.

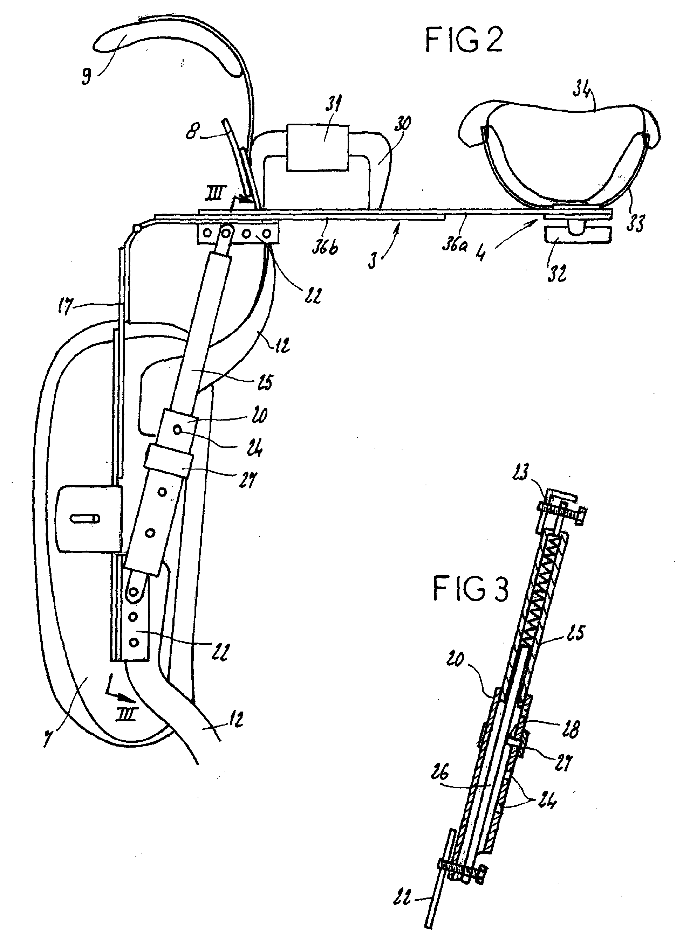

En se référant à la figure 2, on peut voir qu'un montant 17 s'étend verticalement depuis l'une des plaques d'appui latéral 7.

Le support brachial 3 est fixé sur l'extrémité supérieure du montant 17. Une charnière 18 assure l'articulation du support brachial 3 par rapport au corset 2.

Un vérin 20 relie la face inférieure du support brachial 3 au montant 17. Les extrémités du vérin 20 sont fixées respectivement sur une platine 22 solidaire du montant 17 et sur une platine 23 solidaire du support brachial 3. Chacune des platines 22, 23, comprend des perçages permettant de régler l'inclinaison du vérin 20 par rapport à la verticale.

La figure 3 qui représente en coupe le vérin 20, montre que la course de celui-ci peut être réglée. Ce vérin 20 comprend un cylindre 24 dans lequel coulisse un piston 25, une tige 26 assurant le guidage du piston 25.

Plusieurs perçages sont ménagés dans la paroi du cylindre 24. Un cavalier 27 en matériau élastique présentant un ergot 28 s'étendant depuis sa paroi interne vient se placer sur le cylindre 24, l'ergot 28 pénétrant dans l'un des perçages ménagés dans la paroi du cylindre 24.

L'ergot 24 présente une longueur telle que le piston 25 vient en butée contre ce dernier.

Lorsque le cavalier 27 est mis en place sur le cylindre 24 au niveau du perçage le plus éloigné de son point de fixation sur le montant 17, la course du vérin 20 est nulle.

Le support brachial 3 reçoit sur sa face supérieure un berceau 30 pouvant être formé par une bande 31. L'accrochage de la bande 31 sur le berceau 30 est réalisé par un tissu aggripant à boucles et crochets.

Le support brachial 3 comprend deux tiges superposées dont la position relative peut être modifiée pour allonger la longueur totale de celui-ci.

Le support anté-brachial 4 est articulé sur l'extrémité distale du support brachial 3.

Une liaison pivot réalise la jonction entre les supports brachial 3 et anté-brachial 4. Une mollette 32, en fonction de son serrage, permet, soit de bloquer le support anté-brachial 4 dans une position angulaire fixe par rapport au support brachial 3, soit d'autoriser un mouvement en rotation du support anté-brachial 4 par rapport au support brachial 3.

Le support anté-brachial 4 reçoit sur la face supérieure un berceau 33 similaire au berceau 30 dont est muni le support brachial 3.

Une bande 34 recevant un tissu aggripant à boucles et crochets permet d'accrocher la bande 34 sur elle-même obturant ainsi la face ouverte du berceau 33.

Les supports brachial 3 et anté-brachial 4, comprennent chacun deux tiges superposées respectivement 36a, 36b, 37a, 37b et sont téléscopiques grâce à une ouverture oblongue pratiquée dans respectivement les tiges 36a, 37a, permettant un coulissement d'une tige par rapport à l'autre.

L'extrémité distale du support anté-brachial 4 présente une portion inclinée sur laquelle une boule 35 est fixée.

La mise en place du dispositif peut se faire immédiatement après une intervention chirurgicale scapulo-humérale. Dans l'exemple illustré, le dispositif reçoit un membre supérieur droit.

Le bras prend position dans le berceau brachial 30 et l'avant bras dans le berceau anté-brachial 33. Les appuis pubien 6, latéraux 7, sternal 8, viennent en contact du tronc, l'appui huméral 9 venant en contact de l'épaule opposée à celle ayant subie l'intervention. Le réglage de la position des différents appuis ainsi que le réglage de la longueur des supports brachial 3 et anté-brachial 4 permettent une adaptation parfaite du dispositif à la morphologie du patient.

Durant une phase immédiatement post-opératoire pendant laquelle une immobilisation du membre supérieur doit permettre la cicatrisation ligamentaire ou la consolidation osseuse, le membre supérieur est immobilisé dans une position d'abduction, le bras étant sensiblement dans le plan sagital.

Pour maintenir le bras dans cette position, le cavalier 27 permettant de régler la course du vérin 20 est engagé dans le perçage supérieur du cylindre 24, si bien que la course du vérin est nulle.

Il faut noter qu'il est possible de faire varier l'angle d'abduction initial optimal en déplaçant les points de fixation du vérin 20 sur les platines 22, 23 le reliant au montant 17 et au support brachial 3. L'angle d'adbduction initial pourrait être de 90° par rapport à l'axe médian du corps.

La mollette 32 permet de fixer l'orientation angulaire de l'avant bras par rapport au bras.

Quelques jours après l'opération, lorsque le patient peut supporter une rééducation plus dynamique, le cavalier 27 peut être déplacé vers un perçage inférieur autorisant ainsi un mouvement d'abduction adduction sur un arc controlé par un praticien.

Au fur et à mesure de la rééducation, l'arc de mobilité pourra être augmenté en déplaçant le cavalier 24 sur le vérin 27.

En outre, le vérin 24 initialement placé sur le dispositif pourra être remplacé par un vérin présentant un facteur de raideur différent, en vue d'optimiser la rééducation.

L'invention ainsi décrite fournit un dispositif de rééducation scapulo-humérale qui permet d'adapter de façon optimale les phases de rééducation en fonction de chaque patient et permet de restaurer une mobilité entière de l'articulation scapulo-humérale au terme d'une période de rééducation la plus courte possible.

Il va de soi que l'invention n'est pas limitée à la forme de réalisation décrite ci-dessus, mais qu'elle en embrasse au contraire toutes les formes de réalisation définies par les revendications.

## Revendications

1. Dispositif de rééducation scapulo-humérale comprenant un corset (2) sur lequel est articulé un support brachial (3) maintenant un bras dans une position d'abduction, comprenant des moyens de réglage de la position d'abduction optimale initiale et de l'arc de mobilité optimale relient le support brachial (3) au corset (2), **caractérisé en ce qu'**il comprend un vérin (20) à ressort ou à gaz dont une extrémité est fixée sur le corset (2) et dont l'autre extrémité est fixée sur le support brachial (3).

2. Dispositif selon la revendication 1, **caractérisé en ce que** les points de fixation du vérin (20) sont réglables respectivement verticalement par rapport au corset (2) et longitudinalement par rapport au support brachial (3).

3. Dispositif selon la revendication 2, **caractérisé en ce que** le vérin(20) comprend un cylindre (24) dans lequel coulisse un piston (25), le cylindre (24) présentant des perçages dans lesquels est susceptible d'être engagé un ergot (28) formant butée pour le piston (25).

4. Dispositif selon la revendication 3, **caractérisé en ce que** l'engagement de l'ergot (28) dans le perçage du cylindre (24) le plus éloigné de l'extrémité du vérin (20) annule la course du vérin (20).

5. Dispositif selon la revendication 3 ou la revendication 4, **caractérisé en ce qu'**un cavalier (27) en matière élastique susceptible d'être encliqueté sur le cylindre (24) supporte l'ergot (28).

6. Dispositif selon l'une des revendications 2 à 5, **caractérisé en ce que** les moyens de fixation du vérin permettent son interchangeabilité.

7. Dispositif selon l'une des revendication 1 à 6, **caractérisé en ce qu'**une charnière (18) assure l'articulation entre le support brachial et un montant (17) vertical fixé au corset (2).

8. Dispositif selon l'une des revendications 1 à 7, **caractérisé en ce qu'**un support anté-brachial (4) est articulé sur l'extrémité distale du support brachial.

9. Dispositif selon l'une des revendications 1 à 8, **caractérisé en ce que** le corset comprend une plaque d'appui pubien (6), deux plaques d'appui latéral (7), une plaque d'appui sternal (8) et une plaque d'appui huméral (9) reliées par des barres de liaison (12).

10. Dispositif selon la revendication 9, **caractérisé en ce qu'**un appui dorsal (14) est relié par une ceinture souple (15) de manière fixe à l'un des appuis latéraux et de manière amovible à l'autre appui latéral.

11. Dispositif selon la revendication 8, **caractérisé en ce qu'**une boule (35) est disposée à l'extrémité distale du support anté-brachial (4).

## Patentansprüche

1. , Schulterblatt/Oberarm-Rehabilitationsgerät mit einem Korsett (2), an dem eine Armetütze (3) angelenkt ist, die einen Arm in einer Abduktionsposition hält, und mit Mitteln zur Einstellung der optimalen anfänglichen Abduktionsposition und dee optimalen Beweglichkeitsbogens, welche die Armstütze (3) mit dem Korsett (2) verbinden, **dadurch gekennzeichnet, dass** es einen Feder- oder Gas-Stellzylinder (20) aufweist, wovon ein Ende auf dem Korsett (2) und das andere Ende auf der Armstütze (3) befestigt ist.

2. Gerät nach Anspruch 1, **dadurch gekennzeichnet, dass** die Befestigungepunkte des Stellzylinders (20) bezüglich des Korsetts (2) vertikal bzw. bezüglich der Armstütze (3) longitudinal einstellbar sind.

3. Gerät nach Anspruch 2, **dadurch gekennzeichnet, dass** der Stellzylinder (20) einen Zylinder (24) aufweist, in welchem ein Kolben (25) gleitet, wobei der Zylinder (24) Bohrungen aufweist, in die ein Stift (28) eingeführt werden kann, der einen Anschlag für den Kolben (25) bildet.

4. Gerät nach Anspruch 3, **dadurch gekennzeichnet, dass** das Einführen des stiftes (28) in die vom Ende des Stellzylinders (20) am weitesten entfernte Bohrung des Zylinders (24) den Hub des stellzylinders (20) aufhebt.

5. Gerät nach Anspruch 3 oder 4, **dadurch gekennzeichnet, dass** ein Reiter bzw. ein Aufsatz (27) aus elastischem Material, der auf den Zylinder (24) aufgeclipst werden kann, den Stift (28) abstützt.

6. Gerät nach einem der Ansprüche 2 bis 5, **dadurch gekennzeichnet, dass** die Mittel zum Befestigen des Stellzylinders dessen Austauschbarkeit ermöglichen.

7. Gerät nach einem der Ansprüche 1 bis 6, **dadurch gekennzeichnet, dass** ein Scharnier (18) die Anlenkung zwischen der Armstütze und einer an dem Korsett (2) befestigten Vertikalatütze (17) gewährleistet.

8. Gerät nach einem der Ansprüche 1 bis 7, **dadurch gekennzeichnet, dass** eine Unterarmstütze (4) an dem distalen Ende der Armstütze angelenkt ist.

9. Gerät nach einem der Ansprüche 1 bis 8, **dadurch gekennzeichnet, dass** das Korsett eine Schambein-Stützplatte (6), zwei Seiten-Stützplatten (7), eine Brustbein-Stützplatte (8) und eine Oberarm-Stützplatte (9) aufweist, die über Verbindungsstäbe (12) verbunden sind.

10. Gerät nach Anspruch 9, **dadurch gekennzeichnet, dass** eine Rücken-Abstützung (14) über einen flexiblen Gürtel (15) mit der einer Seiten-Abstützung fest und mit der anderen Seiten-Abstützung abnehmbar verbunden ist.

11. Gerät nach Anspruch 9, **dadurch gekennzeichnet, dass** eine Kugel (35) am distalen Ende der Unterarmstütze (4) angeordnet ist.

## Claims

1. Scapulo-humeral rehabilitation device comprising a corset (2) to which there is articulated a brachial support (3) holding an arm in an abducted position, comprising means for setting the initial optimum abduction position and the optimum arc of mobility connecting the brachial support (3) to the corset (2), **characterized in that** it comprises a spring-loaded or gas actuator (20) one end of which is fixed to the corset (2) and the other end of which is fixed to the brachial support (3).

2. Device according to Claim 1, **characterized in that** the points of attachment of the actuator (20) can be adjusted vertically with respect to the corset (2) and longitudinally with respect to the brachial support (3), respectively.

3. Device according to Claim 2, **characterized in that** the actuator (20) comprises a cylinder (24) in which a piston (25) slides, the cylinder (24) having drillings in which a pin (28) can be inserted to form an end stop for the piston (25).

4. Device according to Claim 3, **characterized in that** fitting the pin (28) into the drilling in the cylinder (24) that is furthest from the end of the actuator (20) cancels the travel of the actuator (20).

5. Device according to Claim 3 or Claim 4, **characterized in that** a clip (27) made of elastic material that can be clipped over the cylinder (24) supports the pin (28).

6. Device according to one of Claims 2 to 5, **characterized in that** the actuator attachment means allow the actuator to be interchangeable.

7. Device according to one of Claims 1 to 6, **characterized in that** a hinge (18) provides the joint between the brachial support and a vertical upright (17) fixed to the corset (2).

8. Device according to one of Claims 1 to 7, **characterized in that** an ante-brachial support (4) is articulated to the distal end of the brachial support.

9. Device according to one of Claims 1 to 8, **characterized in that** the corset comprises a pubic support plate (6), two lateral support plates (7), a sternal support plate (8) and a humeral support plate (9) which are connected by connecting bars (12).

10. Device according to Claim 9, **characterized in that** a dorsal support (14) is connected by a flexible belt (15) fixedly to one of the lateral supports and removably to the other lateral support.

11. Device according to Claim 8, **characterized in that** a ball (35) is positioned at the distal end of the antebrachial support (4).
